Europäisches Patentamt

European Patent Office

Office européen des brevets

(19)

(11) Publication number: **0 153 811**

**B1**

(12) **EUROPEAN PATENT SPECIFICATION**

(45) Date of publication of patent specification: **29.06.88**

(51) Int. Cl.⁴: **A 61 F 5/44**

(21) Application number: **85300522.1**

(22) Date of filing: **25.01.85**

(54) **Urine feed to drainage bag.**

(30) Priority: **27.01.84 GB 8402236**

(43) Date of publication of application:
**04.09.85 Bulletin 85/36**

(45) Publication of the grant of the patent:
**29.06.88 Bulletin 88/26**

(84) Designated Contracting States:
**DE FR GB SE**

(56) References cited:
**DE-A-2 601 180**
**DE-A-2 621 018**
**DE-A-2 927 376**
**GB-A-2 103 488**

(73) Proprietor: **BARD LIMITED**
**Pennywell Industrial Estate**
**Sunderland SR4 9EW (GB)**

(72) Inventor: **Harris, Christopher**
**Rose Valley House 3 Wignall Street**
**Lawford Manningtree Essex (GB)**

(74) Representative: **Moon, Donald Keith et al**
**BREWER & SON 5-9 Quality Court Chancery**
**Lane**
**London WC2A 1HT (GB)**

Courier Press, Leamington Spa, England.

## Description

This invention relates to feed of urine to a urine drainage bag and, more particularly, concerns feed of urine through a drip chamber provided in the urine flow path.

A drip chamber is incorporated at the top of a urine drainage bag to create a break in the fluid pathway. This is done to reduce the chances of infection, which may have become established in the urine in the bag, ascending the tube and so infecting the patient's bladder.

United Kingdom patent specification GB—A—2103488 discloses a male incontinence device including a condom retained in position by a retainer ring immediately behind the corona. The condom is pulled inside out over the end of the penis to conduct urine through a connecting cone to a container.

German patent specification DE—A—2927376 discloses a microbe barrier device comprising an antiseptic substance located in a housing in a liquid line. The device is particularly suitable for avoiding retrograde infection when draining body liquids.

A simple drip chamber can fill with urine in certain circumstances, so that the desired break in the fluid feed path is no longer present. This happens when the air in the drip chamber is caused to pass through the non-return valve customarily placed between the drip chamber and the drainage bag itself, located beneath the valve. The air can become transferred either by entrainment in the flow of urine passing through the drip chamber or by allowing the bag to lie flat or become inverted, as may happen when the patient and bag are being transported. Once the drip chamber is filled with urine it remains full because air cannot pass back through the non-return valve into the drip chamber or otherwise enter it, and so the break in the fluid pathway is compromised.

One way of tackling this problem is to introduce an air inlet vent into the drip chamber. This may be either at the end of the urine feed tube nearer the patient or, more usually, at the top of the drip chamber. Now, if the drip chamber contains liquid after the bag is put into its proper upright disposition, air can enter the drip chamber through the vent and so allow the urine to drain into the drainage bag below.

A negative pressure in the space above the liquid level in the drainage bag can arise from the effect of the weight of urine pulling the bag into a shape in which the tension in the bag walls above the urine level tends to pull the opposed walls apart from one another. This negative pressure can draw air through the inlet vent and into the bag. This is undesirable in that the air tends to occupy a part of the volume of the bag which is otherwise available for urine, thus limiting its capacity.

To overcome this disadvantage, it has been proposed to place an outlet vent in the top of the drainage bag itself, to allow the excess air to escape as the bag fills. In normal use, a bag with the two vents identified above works well and maintains a break in the fluid pathway. However, if the bag is laid flat, the drip chamber will fill as soon as the patient produces any fresh flow of urine. If the bag is left lying flat for any appreciable time and if the or each vent includes a hydrophobic bacterial filter membrane, this membrane may eventually "wet-out" i.e. become wetted by the liquid and cease to be air-permeable as required. A further major disadvantage of the vented systems is that the vents represent a significant cost both in terms of materials and in terms of assembly.

As has been demonstrated, vents provide a means of replacing air in the drip chamber should it fill up. The present invention stems from an appreciation that, in the simple closed system as first described, the air from the drip chamber is not lost but simply transferred into the urine storage bag across the non-return valve. It was therefore decided that what was needed was to allow the air in the bag to return to the drip chamber.

In accordance with the invention, there is provided a urine drainage bag to be used in an upright disposition and having a liquid storage chamber to be fed through an aperture in the top of the chamber from a drip chamber above the aperture, itself fed from the top by a urine flow passage from the patient, wherein the drip chamber is vented the aperture is wide enough in use of the bag and upon bringing the drainage bag into its upright disposition to permit air which has passed from the drip chamber to the storage chamber to return through the aperture to the drip chamber and liquid in the drip chamber simultaneously to flow to the storage chamber, and a non-return valve is located on the downstream end of the urine flow passage in the top of the drip chamber.

With the invention, there need be no flow of air into and out of the bag as a whole but only such shuttling of air between the drip chamber and the storage chamber as is consequent upon the handling of the bag. Eliminating the vents reduces complexity and manufacturing costs. The drip chamber may in certain circumstances hold liquid, for example, if the bag is inverted, but the same effect will occur in the prior art bags where the patient is producing a constant or continuing flow of urine, as is often the case with users of these bags, so the risk of infection will be no greater than with the prior bags which have a non-return valve between the drip chamber and the liquid storage chamber.

The present drip chamber can advantageously be made as a simple plastics moulded component by arranging for a moulding die part or core which creates the internal surface of the chamber to be withdrawable from the moulded chamber through its wide outlet aperture. One convenient shape is that of a bell but with the walls diverging only very slightly towards the outlet aperture. This is unlike previous plastics drip chambers for the present drainage bags, which are wider in the middle than at their inlet and outlet ends and so are made as two components subsequently bonded together along a joining line at the widest part of the chamber.

In normal use, the large diameter exit of the drip chamber prevents air being entrained from the chamber into the storage vessel of the drainage bag. There is thus no prospect for the drip chamber to fill by this mechanism. Compressing the storage vessel will not allow urine to pass back from it into the drip chamber because the non-return valve will close to trap the air in the drip chamber and the top of the bag, and this air will compress only very slightly with the pressures that are likely to be encountered in practice. This behaviour is superior to the above-mentioned vented system where compressing the bag closes the non-return valve and sets up a pressure differential across it, so that continued flow of urine only serves to fill up the drip chamber so increasing the risk of transmitting infection to the patient.

Normally, the drainage bags of the invention take the form of two sheets of plastics material welded together around their peripheries. Should such a bag be laid flat then the drip chamber will fill with urine from the storage chamber. However, once the bag is returned to its normal upright position, the drip chamber immediately empties. Besides, if the drip chamber is carefully designed, it need only be angled a little above horizontal when the bag is laid down for air to be trapped in the drip chamber, so ensuring that it does not fill, because the trapped air cannot escape into the storage vessel. One way of so angling the drip chamber, so that its inlet end is held higher than its outlet end, is to provide along the top edge of the bag a handle which projects outwards from one face of the bag, at least when the bag is lying flat on this one face. The projecting part of the handle props up the top edge of the bag and hence the inlet end of the drip chamber.

Furthermore, welds can be provided to join together the two faces of the bag near the outlet of the drip chamber, to pull down the top face of the bag when the bag is lying flat, and so create an impediment to free movement of air from the drip chamber to the storage bag, and hence of liquid from the bag flowing into the drip chamber.

It will be understood that, when the bag is in its normal upright disposition, the handle and welds have no inhibiting effect on the free shuttling of air through the wide outlet end of the drip chamber between the storage bag and the drip chamber.

It will be appreciated that the proposed design holds out a prospect of obtaining all the advantages of even the best vented system but at lower cost. In some situations it may well turn out to be superior.

For a better understanding of the invention, reference will now be made, by way of example, to the accompanying drawings, in which:

Figure 1 is a schematic vertical section of a prior art, unvented, drainage bag;

Figure 2 is a similar section of a prior art vented bag; and

Figure 3 is a similar section of a drainage bag according to the invention.

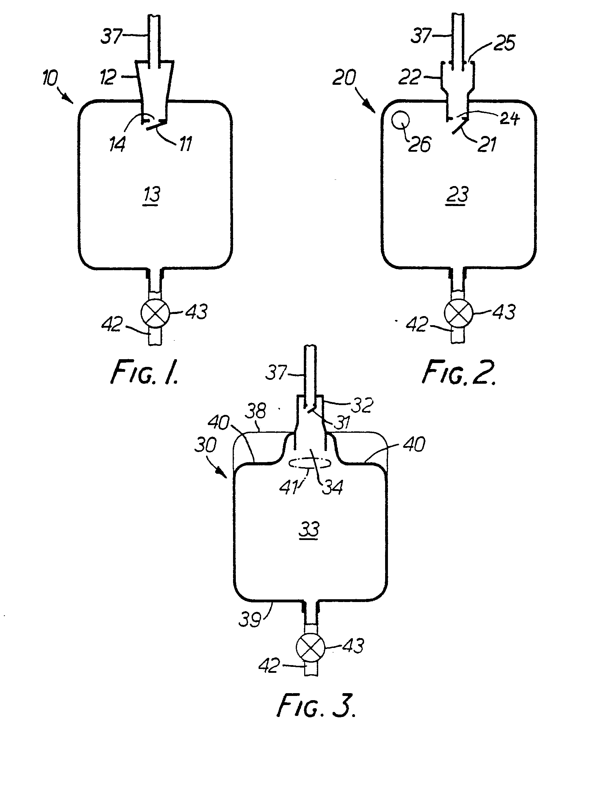

Referring now to Figures 1 and 2, both bags 10 and 20 have a non-return valve 11 and 21 respectively between their drip chamber 12, 22 and their storage chamber 13, 23. Thus, once air has passed from the drip chamber to the storage chamber it cannot normally return through the flow aperture 14, 24 in the non-return valve 11, 21, respectively.

In the Figure 1 embodiment, the outlet aperture 14 is narrow so that the forces of surface tension can restrict flow of liquid and air through the aperture. If the drip chamber for some reason becomes full of liquid, it will tend to remain full of liquid, negating the purpose of the drip chamber. In Figure 2, by contrast, any liquid in the drip chamber 22 is released into the storage chamber 23 by entry of air into the drip chamber 22 through the vent 25.

The air which has flowed into the storage chamber 23 has to be expelled, if the chamber is to hold its full capacity of liquid. It is so expelled, by flow of further liquid into the bag, through the vent 26.

Referring now to Figure 3, the bag 30 has a non-return valve 31 at the top of a drip chamber 32 which itself is separated from a storage chamber 33 by an aperture 34. The non-return valve 31 is between the drip chamber 32 and the urine flow passage 37 from the patient. Conveniently, it is a flap valve constituted by a plastics disc separated from a surrounding ring bonded to the end of the passage 37 by a horse-shoe shaped cut-away portion. The plastics material can be MYLAR (trade mark).

Unless the bag is laid completely flat or with the top edge 38 lower than the bottom edge 39, the air in the drip chamber 32 should remain in this chamber. Specifically, the aperture 34 is wide enough to ensure that, with the bag in the upright disposition shown, flow of urine through the drip chamber 32 cannot entrain the air and carry it through to the storage chamber. If the bag is inverted and then righted, liquid in the drip chamber can change places with air in the storage chamber free of restriction arising from the effects of surface tension which would tend to prevent such interchange through a narrower aperture. For this reason, the aperture is likely to have a diameter which exceeds about 7 or 8 mm.

Casual handling of the bag so that it is laid nearly flat will probably not be sufficient to remove the air break in the liquid infection path to the patient. The performance of the bag in this respect is improved further by configuring the walls of the storage chamber 33 adjacent the aperture 34 such that when the bag is laid flat, air cannot move from the drip chamber or storage chamber to the other chamber except by passing under a low point in the top surface of the storage chamber. This effect is achieved in the illustrated embodiment by welding the opposed storage chamber sheet wall surfaces together along weld lines 40 adjacent the aperture 34, so that air must first flow to a low point 41 if it is to pass from the drip chamber 32 to the storage chamber 33 while the bag 30 is laid flat. The effect of pressure on the contents of the storage chamber 33 will only serve to drive the air more firmly into the drip chamber 32, away from the low point 41.

All the illustrated drainage bags have a urine drain path 42 with a shut-off valve 43. It is by travelling up the path 42 and into the contents of the storage vessel that infection can begin its journey to the patient, so the existence of a continuous liquid path to the patient is less critical before the drainage bag is first emptied than afterwards. The occasions when the drainage bag is most likely to be inverted will tend to occur before, and not after, it is emptied for the first time.

## Claims

1. A urine draining bag (30) to be used in an upright disposition and having a liquid storage chamber (33) to be fed through an aperture (34) in the top of the chamber from a drip chamber (32) above the aperture, itself fed from the top by a urine flow passage (37) from the patient, characterised in that the drip chamber (32) is non-vented, the aperture (34) is wide enough in use of the bag and upon bringing the drainage bag into its upright disposition to permit air which has passed from the drip chamber to the storage chamber to return through the aperture to the drip chamber and liquid in the drip chamber simultaneously to flow to the storage chamber, and a non-return valve (31) is located on the downstream end of the urine flow passage (37), in the top of the drip chamber (32).

2. A bag as claimed in claim 1 characterised in that the valve (31) is a flap valve.

3. A bag as claimed in claim 2 characterised in that the valve (31) is constituted by a plastics disc separated from a surrounding ring bonded to the end of the passage by a horse-shoe shaped cut-away portion.

4. A bag as claimed in any one of the preceding claims characterised in that it has two major faces, each formed from a sheet of plastics material.

5. A bag as claimed in claim 4 characterised in that the faces of the bag are welded together at welds (40) near the said aperture (34) such that, when the bag is lying flat, on one face thereof, the top face is pulled down towards the lower face in the vicinity of the said welds thereby to inhibit flow of air from the drip chamber to the storage chamber while the bag is lying flat.

6. A bag as claimed in claim 4 or 5 characterised in that the bag includes a handle along the top edge (38) of the bag, a part of which projects outwards from one face of the bag, at least when the bag is lying flat on this one face, so that the projecting part of the handle props up the top edge of the bag and hence the inlet end of the drip chamber (32).

7. A bag as claimed in any one of the preceding claims characterised by a drip chamber which is constituted by a single plastics moulding.

## Patentansprüche

1. In einer aufrechten Stellung zu verwendender Urinsammelbehälter (30) mit einer Flüssigkeitssammelkammer (33), die durch eine Öffnung (34) im oberen Bereich der Sammelkammer von einer oberhalb der Öffnung befindlichen Tropfkammer (32) her gefüllt wird, die ihrerseits von oben her durch einen Urinströmungsweg (37) des Patienten gespeist wird, dadurch gekennzeichnet, daß die Tropfkammer (32) unbelüftet ist, daß beim Gebrauch des Urinsammelbehälter und beim Anordnen des Urinsammelbehälters in seiner aufrechten Stellung die Öffnung (34) weit genug ist, um Luft, die von der Tropfkammer in den Sammelbehälter gelangt ist, durch die Öffnung zu der Tropfkammer zurückkehren und gleichzeitig Flüssigkeit aus der Tropfkammer in die Sammelkammer strömen zu lassen, und daß ein Rück-schlagventil (31) am strömungsabwärts gelege-nen Ende des Urinströmungsweges (37) im oberen Bereich der Tropfkammer (32) angeordnet ist.

2. Sammelbehälter nach Anspruch 1, dadurch gekennzeichnet, daß das Ventil (31) ein Klappenventil ist.

3. Sammelbehälter nach Anspruch 2, dadurch gekennzeichnet, daß das Ventil (31) aus einer Kunststoffscheibe gebildet ist, die von einem Umfangsring, der an das Ende des Strömungsweges angeklebt ist, durch einen hufeisenförmigen Ausschnitt getrennt ist.

4. Sammelbehälter nach einem der vorhergehenden Ansprüche, dadurch gekennzeichnet, daß er zwei Hauptseiten aufweist, die jeweils aus einer Kunststoffolie gebildet sind.

5. Sammelbehälter nach Anspruch 4, dadurch gekennzeichnet, daß die Seiten des Sammelbehälters mittels Schweißnähten (40) in der Nähe der Öffnung (34) derart zusammengeschweißt sind, daß, wenn der Behälter flach auf seiner einen Seite liegt, die obenliegende Seite in der Nähe der Schweißnähte nach unten in Richtung der untenliegenden Seite gezogen wird, wodurch ein Luftstrom von der Tropfkammer in die Sammelkammer verhindert wird, während der Sammelbehälter flach liegt.

6. Sammelbehälter nach Anspruch 4 oder 5, dadurch gekennzeichnet, daß der Sammelbehälter einen Griff längs der oberen Kante (38) des Sammelbehälters aufweist, von dem ein Teil wenigstens dann von einer Seite des Sammelbehälters hervorsteht, wenn der Sammelbehälter flach auf dieser einen Seite liegt, so daß der hervorstehende Teil des Griffes die obere Kante des Sammelbehälters und damit das Einlaßende der Tropfkammer (32) aufrechthält.

7. Sammelbehälter nach einem der vorhergehenden Ansprüche, gekennzeichnet durch eine Tropfkammer, die aus einem, einstückigen Kunststofformteil besteht.

**Revendications**

1. Poche (30) d'évacuation d'urine destinée à être utilisée dans une position verticale et possédant une chambre de stockage de liquide (33) destinée à être alimentée à travers une ouverture (34) située au sommet de la chambre à partir d'une chambre de déversement (32) située au-dessus de l'ouverture, ellemême alimentée par le malade au niveau de son sommet à l'aide d'un conduit d'écoulement d'urine (37), caractérisée en ce que la chambre de déversement (32) est non ventilée, et en ce que l'ouverture (34) est assez large lors de l'utilisation de la poche et lorsque l'on met la poche d'évacuation dans sa position verticale pour permettre à l'air qui a passé de la chambre de déversement à la chambre de stockage de retourner dans la chambre de déversement à travers l'ouverture et au liquide situé dans la chambre de déversement de s'écouler simultanément vers la chambre de stockage, et en ce qu'une soupape de non retour (31) est située sur l'extrémité aval du conduit d'écoulement d'urine (37), au sommet de la chambre de déversement (32).

2. Poche selon la revendication 1, caractérisée en ce que la soupape (31) est une soupape à clapet.

3. Poche selon la revendication 2, caractérisée en ce que la soupape (31) est constituée par un disque en matière plastique séparé par une partie découpée en forme d'étrier d'un anneau qui l'entoure et fixé à l'extrémité du conduit.

4. Poche selon l'une quelconque des revendications précédentes, caractérisée en ce qu'elle possède deux faces principales, chacune constituée d'une feuille de matière plastique.

5. Poche selon la revendication 4, caractérisée en ce que les faces de la poche sont soudées entre elles selon des soudures (40) situées à proximité de l'ouverture (34) de façon telle que lorsque la poche est mise à plat sur l'une de ses faces, la face supérieure est tirée vers le bas vers la face inférieure au voisinage desdites soudures de façon à empêcher l'écoulement de l'air à partir de la chambre de déversement vers la chambre de stockage lorsque la poche est à plat.

6. Poche selon les revendications 4 ou 5, caractérisée en ce que la poche comporte une poignée située le long du bord supérieur (38) de la poche, dont une partie fait saillie vers l'extérieur à partir d'une face la poche, au moins lorsque la poche est à plat sur cette face, de façon à ce que la partie en saillie de la poignée relève vers le haut le bord supérieur de la poche et par conséquent l'extrémite d'entrée de la chambre de déversement (32).

7. Poche selon l'une quelconque des revendications précédentes, caractérisée en ce que la chambre de déversement est constituée par une seule pièce de matière plastique moulée.

0 153 811

37
12
10
14
11
13
42
43

FIG. 1.

37
25
22
24
21
20
26
23
42
43

FIG. 2.

37
32
38
31
40
30
40
41
34
33
39
43
42

FIG. 3.

1